# EUROPEAN PATENT SPECIFICATION

(11) **EP 0 315 683 B1**
(45) Date of publication and mention of the grant of the patent: **03.02.1999**
(21) Application number: 88905552.1
(22) Date of filing: 03.06.1988
(51) Int. Cl.: C12P 21/00, C12N 5/00, C12N 1/00

(54) **DNA SEQUENCES, RECOMBINANT DNA MOLECULES AND PROCESSES FOR PRODUCING LYMPHOCYTE FUNCTION ASSOCIATED ANTIGEN-3**
DNS SEQUENZEN, REKOMBINANTE DNS-MOLEKÜLE SOWIE VERFAHREN ZUR HERSTELLUNG DES AN DER LYMPHOCYTEN-FUNKTION ASSOZIIERTEN ANTIGENS-3
SEQUENCES ADN, MOLECULES ADN RECOMBINANT, ET PROCEDES DE PRODUCTION DE L'ANTIGENE-3 ASSOCIE A LA FONCTION LYMPHOCYTE

(30) Priority: 03.06.1987 US 57615
(43) Date of publication of application: 17.05.1989
(73) Proprietor: BIOGEN, INC., Cambridge Massachusetts 02142 (US); DANA-FARBER CANCER INSTITUTE, INC., Boston, MA 02115 (US)
(72) Inventor: WALLNER, Barbara P., Cambridge, MA 02139 (US); SPRINGER, Timothy A., Newton, MA 02167 (US); HESSION, Catherine, South Weymouth, MA 02190 (US); TIZARD, Richard, Newton, MA 02190 (US); MATTALIANO, Robert, San Carlos, CA 94070 (US); DUSTIN, Michael L., Boston, MA 02215 (US)
(74) Representative: Bannerman, David Gardner
(86) International application number: PCT/US88/01924
(87) International publication number: WO 88/09820

(56) References cited:
- US-A- 4 738 927
- THE JOURNAL OF IMMUNOLOGY, vol. 136, no. 8, 15th April 1986, pages 3085-3091, The American Association of Immunologists; J.A. BARBOSA et al.: "Gene mapping and somatic cell hybrid analysis of the role of human lymphocyte function-associated antigen-3 (LFA-3) in CTL-target interactions"
- PROC. NATL. ACAD. SCI. USA, vol. 84, no. 10, May 1987, pages 3365-3369; B. SEED et al.: "Molecular cloning of the CD2 antigen, the T-cell erythrocyte receptor, by a rapid immunoselection procedure"
- J. of Exper. Med., Vol. 166, issued 1987 Oct. (Amsterdam, Netherlands), (WALLNER et al.), Primary Structure of Lymphocyte Function Associated Antigen 3 (LFA-3) see pgs 923-931.
- J. of Exp. Med., Vol. 165, issued 1987 March (Amsterdam, Netherlands), (DUSTIN et al), Purified Lymphocyte Function Associated Anigen 3 Binds to CD2 and Mediates T Lymphocyte Adhesion see pgs. 677-692.
- Proc. Natl. Acad. Sci., Vol. 83, issued Nov 1986, (Washington, D.C) Molecular "Cloning of the Human T-Lymphocyte Surface CD2(TII) Antigen" see pgs 8718-8722, see particularly page 8719.
- J. of Exper. Med., Vol. 166, issued Oct. 1987, (Amsterdam, Netherlands), (SELVARAJ et al.), "Deficiency of Lymphocyte Function Associated Antigen 3 (LFA3) in Paroxysmal Nocturnal Hemoglobinuria" see pages 1011-1025. see particularly pages 1011, 1012.
- Proc. Natl. Acad. Sci. Vol. 79 issued Dec. 1982 (Washington, D.C.), (SANCHEZ-MADRID et al), "Three Distinct Antigens Associated with Human T-Lymphocytes Mediated Cytolysis LFA-1, LFA-2, LFA-3" pages 7489-7493.
- Nature, Vol. 329, issued 29 Oct. 1987 (Tokyo, Japan), (SEED), "An LFA-3 cDNA Encodes a Phospholipid Linked Membrane Protein Homologous to its Receptor CD2" pages 840-844.
- Proc. Natl. Acad. Aci. Vol. 81, issued Jan, 1984 (Washington, D.C.) "Immunoprecipitation of Cell Surface Structures of Cloned Cytotoxic T Lymphocytes by Clone Specific Antisera pages 573-577.
- J. of Immunology, Vol. 138, 1 June 1987 (Washington, D.C.), (MAKGOBA et al.) "Human T Cell Rosetting is Mediated by LFA3 on Erythrocytes" pages 3587-3589.

## Description

This invention relates to DNA sequences, recombinant DNA molecules and processes for producing Lymphocyte Function Associated Antigen-3 (LFA-3). More particularly, the invention relates to DNA sequences that are characterized in that they code on expression in an appropriate unicellular host for LFA-3 or derivatives thereof that bind to CD2, the receptor on the surface of T-lymphocytes. More preferably, the LFA-3 of this invention and its derivatives bind to CD2 on the surface of T-lymphocytes. Most preferably, they also inhibit adhesion between T-lymphocytes and target cells. In accordance with this invention, unicellular hosts transformed with these DNA sequences and recombinant DNA molecules containing them may also be employed to produce LFA-3 essentially free of other proteins of human origin. This novel antigen may then be used in the therapeutic and diagnostic compositions and methods of this invention.

### BACKGROUND OF THE INVENTION

T-lymphocytes play a major role in the immune response by interacting with target and antigen presenting cells. For example, the T-lymphocyte mediated killing of target cells is a multi-step process involving adhesion of a cytolytic T-lymphocyte to a target cell. And, helper T-lymphocytes initiate the immune response by adhesion to antigen-presenting cells.

These interactions of T-lymphocytes with target and antigen-presenting cells are highly specific and depend on the recognition of an antigen on the target or antigen-presenting cell by one of the many specific antigen receptors on the surface of the T-lymphocyte.

The receptor-antigen interaction of T-lymphocytes and other cells is also facilitated by various T-lymphocyte surface proteins, e.g., the antigen receptor complex CD3(T3) and accessory molecules CD4, LFA-1, CD8, and CD2. It is also dependent on accessory molecules, such as LFA-3, ICAM-1 and MHC that are expressed on the surface of the target or antigen-presenting cells. In fact, it is hypothesized that the accessory molecules on the T-lymphocytes and on the target or antigen-presenting cells interact with each other to mediate intercellular adhesion. Accordingly, these accessory molecules are thought to enhance the efficiency of lymphocyte-antigen-presenting cell and lymphocyte-target cell interactions and to be important in leukocyte-endothelial cell interactions and lymphocyte recirculation.

For example, recent studies have suggested that there is a specific interaction between CD2 (a T-lymphocyte accessory molecule) and LFA-3 (a target cell accessory molecule) that mediates T-lymphocyte adhesion to the target cell. This adhesion is essential to the initiation of the T-lymphocyte functional response (M. L. Dustin et al., "Purified Lymphocyte Function-Associated Antigen-3 Binds To CD2 And Mediates T Lymphocyte Adhesion, J. Exp. Med., 165, pp. 677-92 (1987); Springer et al., Ann. Rev. Immunol. (1987) (in press)). And, monoclonal antibodies to either LFA-3 or CD2 have been shown to inhibit a spectrum of cytolytic T lymphocyte and helper T lymphocyte dependent responses (F. Sanchez-Madrid et al., "Three Distinct Antigens Associated With Human T-Lymphocyte-Mediated Cytolysis: LFA-1, LFA-2 , And LFA-3", Proc. Natl. Acad. Sci. USA, 79, pp. 7489-93 (1982)).

LFA-3 is found on antigen-presenting cells, and target cells, specifically on monocytes, granulocytes, CTL's, B-lymphoblastoid cells, smooth muscle cells, vascular endothelial cells, and fibroblasts (Springer et al., supra).

Human LFA-3 has been purified from human erythrocytes (Dustin et al., supra). It is a glycoprotein of 60,000 to 70,000 molecular weight, having a sugar content of about 50%. This purified LFA-3 binds to CD2 on the surface of T-lymphocytes and inhibits adhesion between T-lymphocytes and erythrocytes (Dustin et al., supra).

However, for ultimate use in therapy and diagnosis larger amounts of less costly LFA-3 are required than would be available from purification from erythrocytes. Moreover, for therapeutic use it would be more preferable to obtain LFA-3 from a source other than human erythrocytes, which may be contaminated with viruses, such as hepatitis B viruses or AIDS viruses.

Makgoba et al (J Immunol., 1987, 138, 3587-3589), Barbosa et al (J Immunol., 1986, 136, 3085-3091) and Seed et al (PNAS, 1987, 84, 3365-3369) disclose further studies of CD2 antigen and LFA-3. In particular LFA-3 antibodies are disclosed.

### SUMMARY OF THE INVENTION

The invention provides a DNA sequence selected from the group consisting of:
(a) the DNA insert carried in phage λHT16 (ATCC 75107) - formerly IVI 10133);
(b) DNA sequences which hybridize under conditions equivalent to about 20° to 27° below Tm and 1 M sodium chloride to the LFA-3 DNA sequence of (a) and which code on expression for a polypeptide that binds to CD2, the receptor on the surface of T-lymphocytes; and
(c) DNA sequences which are degenerate to any of the DNA sequences of (a) and (b) and code on expression for the same polypeptides.

A further aspect of the invention provides polypeptides according to claim 12 and methods and uses of such polypeptides.

### BRIEF DESCRIPTION OF THE DRAWINGS

Figure 1 depicts the amino acid sequences of the N-terminal and various peptide fragments of human LFA-3, purified from human erythrocytes using immunoaffinity chromatography.

Figure 2 depicts two pools of chemically synthesized oligonucleotide DNA probes derived from the amino acid sequence of human LFA-3 purified from human erythrocytes.

Figure 3 depicts the DNA sequence of the DNA insert carried in phage AHT16. Figure 3 also depicts the nucleotide sequence of a cDNA sequence coding on expression for human LFA-3 and the amino acid sequence deduced therefrom.

Figure 4 depicts the relevant portions of sequencing plasmid pNN01.

Figure 5 depicts the nucleotide sequence of probes LF-10, LF-11, NN-A, NN-B, NN-C, and NN-D.

### DETAILED DESCRIPTION OF THE INVENTION

We isolated the DNA sequences of this invention from two libraries: a λgtl0 cDNA library derived from peripheral blood lymphocytes from leukophoresis #9 and a λgtl0 cDNA library derived from human tonsil. However, we could also have employed libraries prepared from other cells that express LFA-3. These include, for example, monocytes, granulocytes, CTL's, B-lymphoblastoid cells, smooth muscle cells, vascular endothelial cells and fibroblasts. We also could have used a human genomic bank.

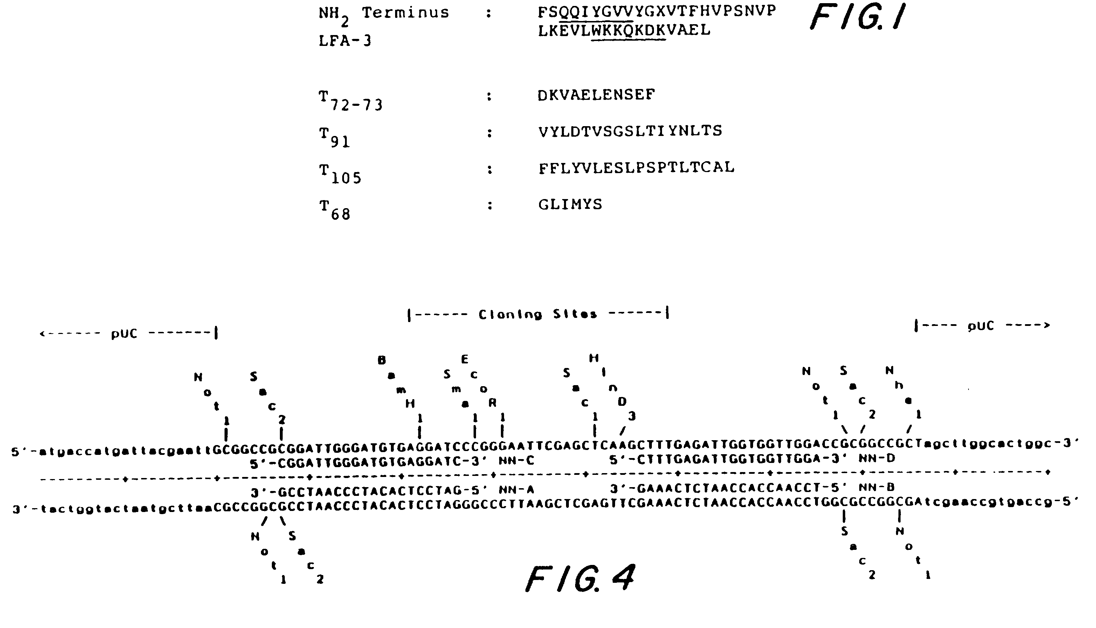

For screening these libraries, we used a series of chemically synthesized anti-sense oligonucleotide DNA probes. We selected these probes from a consideration of the amino acid sequences of various fragments of LFA-3 that we determined using LFA-3 purified from human erythrocytes. These fragments are depicted in Figure 1. We selected amino acids from various areas of LFA-3 that permitted the construction of oligonucleotide probes of minimal degeneracy.

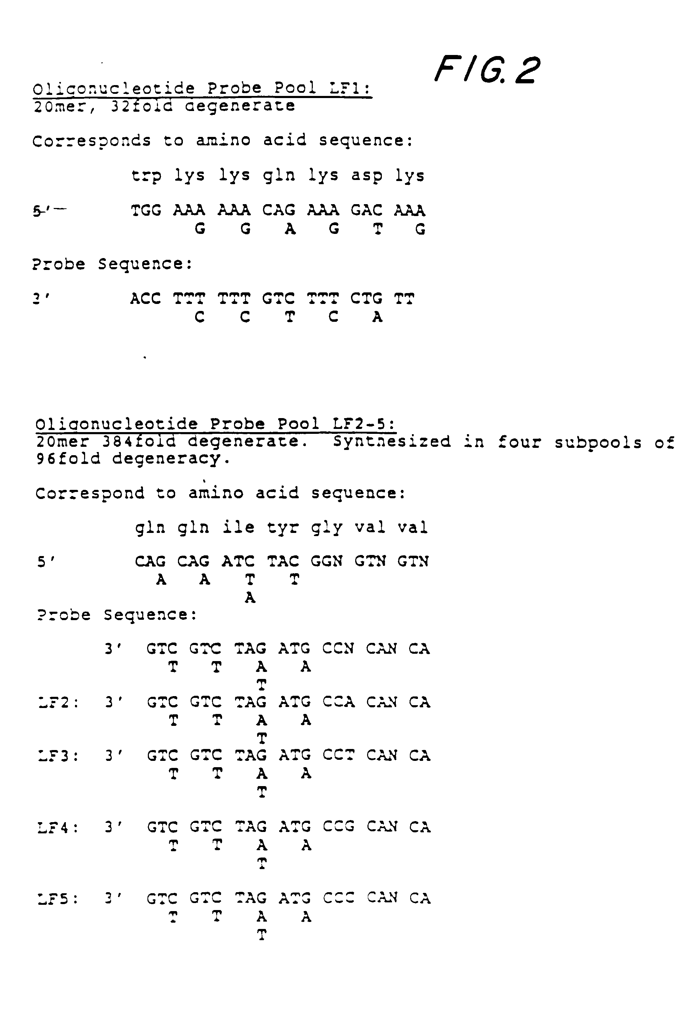

We prepared two pools of probes: LF1 and LF2-5. These pools are depicted in Figure 2. LF1 is a 32-fold degenerate 20-mer and LF2-5 is a 384-fold degenerate 20-mer. Because of the high degeneracy of this latter pool, we subdivided the pool into four subpools -- LF2, LF3, LF4 and LF5 -- of 96-fold degeneracy each.

For screening, we hybridized our oligonucleotide probes to our cDNA libraries utilizing a plaque hybridization screening assay. We selected clones hybridizing to several of our probes. And, after isolating and subcloning the cDNA inserts of the selected clones into plasmids, we determined their nucleotide sequences and compared the amino acid sequences deduced from those nucleotide sequences to the amino acid sequences that we had determined previously from our purified human LFA-3. As a result of these comparisons, we determined that all of our selected clones were characterized by cDNA inserts coding for amino acid sequences of human LFA-3.

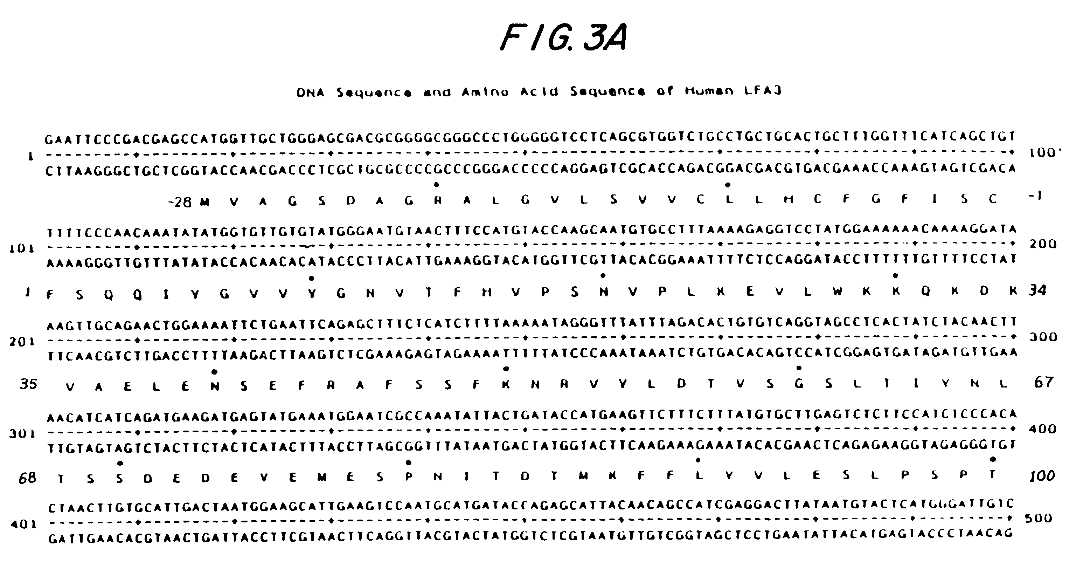

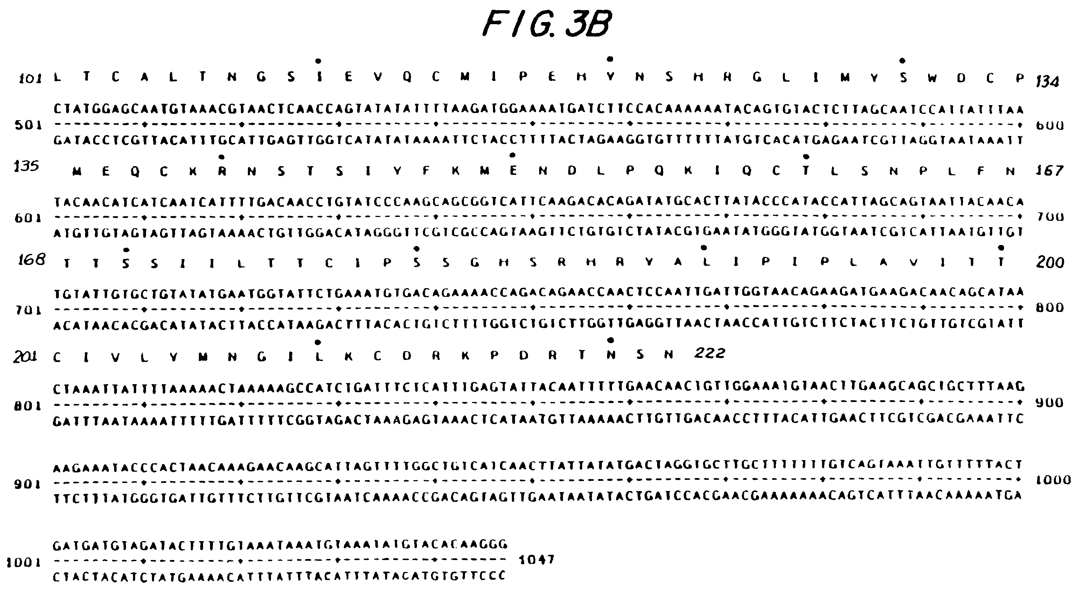

We have depicted in Figure 3 the nucleotide sequence of the longest of these cDNA inserts (the DNA insert of phage λHT16) and the DNA sequence coding for LFA-3 and the amino acid sequence deduced therefrom. As shown in Figure 3, this cDNA insert has an open reading frame of 750 bp (250 amino acids), a 16 bp 5' untranslated region and a 201 bp 3' untranslated region. A comparison of the deduced amino acid sequence of Figure 3 with the N-terminal amino acid sequence that we determined from LFA-3 purified from human erythrocytes suggests that amino acids -28 to -1 comprise a signal sequence and that amino acids 1 to 222 comprise the protein sequence of mature LFA-3.

The cDNA sequence depicted in Figure 3 and contained in deposited clone λHT16 may be used in a variety of ways in accordance with this invention. It, portions of it, or synthetic or semi-synthetic copies of them, may be used as DNA probes to screen other human or animal cDNA or genomic libraries to select by hybridization other DNA sequences that are related to LFA-3. Typically, conventional hybridization conditions, e.g., about 20° to 27°C below Tm are employed in such selections. However, less stringent conditions may be necessary when the library is being screened with a probe from a different species than the library, e.g., the screening of a mouse library with a human probe.

The cDNA sequence of Figure 3, portions of it, or synthetic or semi-synthetic copies of them, may also be used as starting materials to prepare various mutations. Such mutations may be either degenerate, i.e., the mutation does not change the amino acid sequence encoded for by the mutated codon, or non-degenerate, i.e., the mutation changes the amino acid sequence encoded for by the mutated codon. Both types of mutations may be advantageous in producing or using LFA-3's of this invention. For example, these mutations may permit higher levels of production, easier purification or higher LFA-3 activity.

For all of these reasons, the DNA sequences of this invention are selected from the group consisting of:
(a) the DNA sequence: the DNA insert carried in phage λHT16;
(b) DNA sequences which hybridize under conditions equivalent to about 20° to 27°C below Tm to the aforementioned DNA sequence and which code on expression for a polypeptide that binds to CD2, the receptor on the surface of T-lymphocytes; and
(c) DNA sequences which are degenerate to any of the sequences of(a) and (b) and code on expression for the same polypeptide.

Preferably, the DNA sequences of this invention code for a polypeptide having the sequence depicted for amino acids 1 to 222 of Figure 3 (with or without an additional N-terminal methionine) or portions thereof. More preferably, the DNA sequence for this invention will be modified as compared to that of Figure 3 (amino acids 1 to 222) in order to remove from it those portions that code for the hydrophobic transmembrane portion, e.g., from about nucleotide 662 to 715, to allow production of a smaller protein. Most preferably, the DNA sequences of this invention code for soluble proteins or peptides that bind to CD2, the receptor on the surface of T-lymphocytes. More preferably, the LFA-3 and derivatives of this invention bind to CD2 on the surface of T-lymphocytes. Most preferably, they also inhibit adhesion between T-lymphocytes and target cells.

The DNA sequences of this invention are also useful for producing the LFA-3 or its derivatives coded on expression by them in unicellular hosts transformed with those DNA sequences. As well known in the art, for expression of the DNA sequences of this invention the DNA sequence should be operatively linked to an expression control sequence in an appropriate expression vector and employed in that expression vector to transform an appropriate unicellular host.

Such operative linking of a DNA sequence of this invention to an expression control sequence, of course, includes the provision of a translation start signal in the correct reading frame upstream of the DNA sequence. If the particular DNA sequence of this invention being expressed does not begin with a methionine, e.g., mature LFA-3 which begins with a phenylalanine, the start signal will result in an additional amino acid -- methionine -- being located at the N-terminus of the product. While such methionyl-containing-product may be employed directly in the compositions and methods of this invention, it is usually more desirable to remove the methionine before use. Methods are available in the art to remove such N-terminal methionines from polypeptides expressed with them. For example, certain hosts and fermentation conditions permit removal of substantially all of the N-terminal methionine in vivo. Other hosts require in vitro removal of the N-terminal methionine. However, such in vivo and in vitro methods are well known in the art.

A wide variety of host/expression vector combinations may be employed in expressing the DNA sequences of this invention. Useful expression vectors, for example, may consist of segments of chromosomal, non-chromosomal and synthetic DNA sequences, such as various known derivatives of SV40 and known bacterial plasmids, e.g., plasmids from E.coli including col E1, pCR1, pBR322, pMB9 and their derivatives, wider host range plasmids, e.g., RP4, phage DNAs, e.g., the numerous derivatives of phage A, e.g., NM989, and other DNA phages, e.g., M13 and Filamenteous single stranded DNA phages, yeast plasmids such as the 2µ plasmid or derivatives thereof, and vectors derived from combinations of plasmids and phage DNAs, such as plasmids which have been modified to employ phage DNA or other expression control sequences. For animal cell expression, we prefer to use plasmid BG312, a plasmid containing the major late promoter of adenovirus 2.

In addition, any of a wide variety of expression control sequences -- sequences that control the expression of a DNA sequence when operatively linked to it -- may be used in these vectors to express the DNA sequence of this invention. Such useful expression control sequences, include, for example, the early and late promoters of SV40 or the adenovirus, the lac system, the trp system, the TAC or TRC system, the major operator and promoter regions of phage λ, the control regions of fd coat protein, the promoter for 3-phosphoglycerate kinase or other glycolytic enzymes, the promoters of acid phosphatase, e.g., Pho5, the promoters of the yeast α-mating factors, and other sequences known to control the expression of genes of prokaryotic or eukaryotic cells or their viruses, and various combinations thereof. For animal cell expression, we prefer to use an expression control sequence derived from the major late promoter of adenovirus 2.

A wide variety of unicellular host cells are also useful in expressing the DNA sequences of this invention. These hosts may include well known eukaryotic and prokaryotic hosts, such as strains of E.coli, Pseudomonas, Bacillus, Streptomyces, fungi, such as yeasts, and animal cells, such as CHO and mouse cells, African green monkey cells, such as COS1, COS7, BSC1, BSC40, and BMT10, and human cells and plant cells in tissue culture. For animal cell expression, we prefer mouse L-cells.

It should of course be understood that not all vectors and expression control sequences will function equally well to express the DNA sequences of this invention. Neither will all hosts function equally well with the same expression system. However, one of skill in the art may make a selection among these vectors, expression control sequences, and hosts without undue experimentation and without departing from the scope of this invention. For example, in selecting a vector, the host must be considered because the vector must replicate in it. The vector's copy number, the ability to control that copy number, and the expression of any other proteins encoded by the vector, such as antibiotic markers, should also be considered.

In selecting an expression control sequence, a variety of factors should also be considered. These include, for example, the relative strength of the system, its controllability, and its compatibility with the particular DNA sequence, of this invention, particularly as regards potential secondary structures. Unicellular hosts should be selected by consideration of their compatibility with the chosen vector, the toxicity of the product coded on expression by the DNA sequences of this invention to them, their secretion characteristics, their ability to fold proteins correctly, their fermentation requirements, and the ease of purification of the products coded on expression by the DNA sequences of this invention.

Within these parameters one of skill in the art may select various vector/expression control system/host combinations that will express the DNA sequences of this invention on fermentation or in large scale animal culture, e.g., mouse L cells.

The polypeptides produced on expression of the DNA sequences of this invention may be isolated from the fermentation or animal cell cultures and purified in a variety of ways well known in the art. Such isolation and purification techniques depend on a variety of factors, such as how the product is produced, whether or not it is soluble or insoluble, and whether or not it is secreted from the cell or must be isolated by breaking the cell. One of skill in the art, however, may select the most appropriate isolation and purification techniques without departing from the scope of this invention.

The polypeptides produced on expression of the DNA sequences of this invention, e.g., met-LFA-3 (amino acids 1-222 of Figure 3) or LFA-3 (amino acids 1-222 of Figure 3), the preferred smaller less hydrophobic derivatives thereof, or the more preferred soluble derivatives thereof, are essentially free of other proteins of human origin and are not contaminated by viruses, such as hepatitis B virus and AIDS. Thus, they are different than LFA-3 purified from human erythrocytes.

These polypeptides are useful in compositions and methods to block or to augment the immune response. For example, the polypeptides of this invention are active in inhibiting cytolytic T-lymphocyte activity by interfering with their interaction with target cells. They have a similar effect on the immune response because they interfere with the interaction of helper T-cells and antigen-presenting cells. Furthermore, the compounds of this invention may be used to target specific T cells for lysis and immune suppression or to deliver drugs, such as lymphokines, to the specifically targeted T-cells. More preferably, soluble derivatives of the polypeptides of this invention may be employed to saturate the CD2 sites of T-lymphocytes thus inhibiting T-cell activation. This effect is plainly of great utility in graft-vs-host disease, in autoimmune diseases, e.g., rheumatoid arthritis, and in preventing allograft rejection. Furthermore, the polypeptides of this invention are preferred over monoclonal antibodies to LFA-3 or CD2 because the polypeptides of this invention are less likely to elicit immune responses in humans than are antibodies raised in species other than humans. The therapeutic compositions of this invention typically comprise an immunosuppressant or enhancement effective amount of such polypeptide and a pharmaceutically acceptable carrier. The therapeutic methods of this invention comprise the steps of treating patients in a pharmaceutically acceptable manner with those compositions.

The compositions of this invention for use in these therapies may be in a variety of forms.
These include, for example, solid, semi-solid and liquid dosage forms, such as tablets, pills, powders, liquid solutions or suspensions, liposomes, suppositories, injectable and infusable solutions. The preferred form depends on the intended mode of administration and therapeutic application. The compositions also preferably include conventional pharmaceutically acceptable carriers and adjuvants which are known to those of skill in the art. Preferably, the compositions of the invention are in the form of a unit dose and will usually be administered to the patient one or more times a day.

Generally, the pharmaceutical compositions of the present invention may be formulated and administered using methods and compositions similar to those used for other pharmaceutically important polypeptides (e.g., alpha-interferon). Thus, the polypeptides may be stored in lyophilized form, reconstituted with sterile water just prior to administration, and administered by the usual routes of administration such as parenteral, subcutaneous, intravenous or intralesional routes.

The polypeptides of this invention or antibodies against them are also useful in diagnostic compositions and methods to detect T-cell subsets or CD2+ cells or to monitor the course of diseases characterized by excess or depleted T-cells, such as autoimmune diseases, graft versus host diseases and allograft rejection.

Finally, the polypeptides of this invention or antibodies against them are useful in separating B and T cells. For example, when bound to a solid support the polypeptides of this invention or antibodies to them will separate B and T cells.

In order that this invention may be better understood, the following examples are set forth. These examples are for purposes of illustration only, and are not to be construed as limiting the scope of the invention in any manner.

### EXAMPLES

### Purification and Sequencing of Human LFA-3:

We purified the human LFA-3 that we employed to generate tryptic fragments and to determine the partial amino acid sequences depicted in Figure 1 from outdated human erythrocytes (obtained from American Red Cross, Needham, Massachusetts) using a modification to procedure of Dustin et al., supra. For completeness that modified purification procedure is described below.

We purified monoclonal antibody (MAb) TS2/9 (Sanchez-Madrid et al., supra) from hybridoma culture supernatants by (NH₄)₂SO₄ precipitation and protein A affinity chromatography. We then used this purified MAb to prepare affinity columns for use in the following purification.

We coupled purified MAb to Sepharose CL-4B by a modification of the method of Cuatrecasas (March et al., Anal. Biochem., 60, p. 149 (1974)). We activated washed Sepharose CL-4B (Pharmacia, Upsala, Sweden) with 40 mg/ml CNBr in 1 M Na₂CO₃ for 10 min on ice and then washed it with distilled water and 0.1 mM HCl. We filtered the activated Sepharose to a moist cake and added it to the purified antibody solution with 2-4 mg/ml IgG (LFA-3 MAb or mouse IgG) in 0.05 M NaCl and 0.1 M NaHCO₃ (pH 8.4). We then mixed the suspension end over end for 20 h and then blocked any remaining reactive groups by addition of ethanolamine to 50 mM and incubation for 1 h. We checked the supernatant for uncoupled antibody by measuring absorbance at 280 nm. Coupling was usually on the order of 90%. We then poured the MAb-coupled Sepharose into a column and washed it with one cycle of pH 11 and pH 3 buffers (see below) before use for affinity chromatography.

We purified the LFA-3 after solubilization with Triton X-100 by affinity chromatography as previously described (Dustin et al., supra). We performed all steps at 4°C.

We obtained outdated human erythrocytes from the American Red Cross (Needham, Massachusetts). We washed cells from 2 units of whole blood 3 times with PBS (pH 7.2). We then pelleted the packed cells to about 500 ml and added another 500 ml of PBS (pH 7.2) with 2% Triton X-100, 1 mM phenylmethylsulfonyl fluoride, 5 mM iodoacetamide and 0.2 trypsin inhibitor units/ml aprotinin to the red cell suspension while stirring. After 1 h we centrifuged the lysate at 150,000g for 2 h. We then passed the cleared lysate over two columns in series at a flow rate of 20 ml/h: (1) a mouse IgG-Sepharose column (2 ml at 2 mg/ml) to absorb some contaminants and to filter out any particulate material, and (2) an LFA-3 MAb-Sepharose column (5-10 ml at 2 mg/ml). We washed the LFA-3 MAb-Sepharose column with 5 column volumes of 50 mM sodium phosphate (pH 7.2), 0.25 M NaCl, 0.1% Triton X-100, then 5 column volumes of 20 mM triethylamine (pH 11), 0.25 M NaCl, 0.1% Triton X-100 and again with 2 column volumes of the pH 7.2 buffer, all at a flow rate of 1 ml/min. We then eluted the remaining bound LFA-3 with 5 column volumes of 50 mM glycine HC1 (pH 3), 0.25 M NaCl, 0.1% Triton X-100 at a flow rate of 20 ml/h. We neutralized the eluted LFA-3 by collecting into 0.1 volume of 1M Tris-HCl (pH 8.6), 0.1% Triton X-100.

When we used octyl-β-D-glucopyranoside (OG) (Calbiochem) to elute LFA-3, all steps were the same until after the high pH wash. At that point we washed the column with 5 volumes of phosphate buffer (pH 7.2), 0.15 M NaCl, containing 1% OG, and eluted the LFA-3 using glycine buffer (pH 3), 0.15 M NaCl, 1% OG. The elution profile was similar to that obtained with Triton X-100. We followed our LFA-3 purification in a semiquantitative manner using a "dot blot" assay (Hawkes et al., Anal. Biochem., 119, p. 142 (1982) with¹²⁵-LFA-3 MAb.

To prepare the purified LFA-3 for trypsin digestion and amino acid sequencing, we deglycosylated it using N-glycosidase F, according to the supplier's instructions with minor modifications (Genzyme, Boston Massachusetts). We first separated the LFA-3 from the detergent by ethanol precipitation (Triton X-100) or ultrafiltration (OG). We then denatured the LFA-3 (1 nmol) by boiling with 25 µl of 0.5% SDS, 0.2 M 2-mercaptoethanol for 5 min. We next treated the denatured LFA-3 with 5 U/nmole N-glycanase in 50 mM Tris-HCl (pH 8.6) with 3% Triton X-100 and 10 mM 1,10-phenanthroline for 20 h at 37°C in a final volume of 50 µl and precipitated the deglycosylated LFA-3 (NG-LFA-3) in ethanol (5 vol. 100% ethanol overnight at -20°C).

We prepared highly purified N-glycanase treated LFA-3 (25 kd) by preparative SDS-PAGE and electroelution. We reduced N-glycanase-treated LFA-3 (approximately 350 pmoles) with 5mM DTT in 250 mM n-ethylmorpholine acetate, 1 mM EDTA, 0.2% SDS (pH 8.25) under argon for 5 min at 100°C, and alkylated it with 11 mM sodium iodoacetate for 30 min at 23°C in the dark. We desalted the product (CM-NG-LFA-3) by gel filtration on Sephadex G-25 M equilibrated with 0.2% SDS, 250 mM N-ethylmorpholinoacetate (pH 8.25) and lyophilized it. We then dispersed the lyophilized LM-NG-LFA-3 in 10 ml absolute ethanol, stored at -20°C for 7 days, and recovered the LFA-3 by centrifugation at 6000 x g for 30 min at 4°C. We dissolved the pellet in 400 µl of 0.1 M NH₄HCO₃ and digested it with TPCK-trypsin (15% w/w) as described in Pepinsky et al., JBC, 261, p. 4239 (1986). We acidified the digest with formic acid to a final concentration of 20% formic acid and immediately fractionated the fragments by reverse phase high pressure liquid chromatography on a narrow bore C₈ column (Aquapore RP300, 0.21 x 10 cm; Brownlee Fabs). We eluted the resulting peptides with a gradient of acetonitrile (0-75%) in 0.1% trifluoroacetic acid at a flow rate of 0.3 ml/min (0.5 min fractions being collected). We monitored the column eluates at both 214 and 280 nm.

We subjected each of intact LFA-3 (300 pMoles), NG LFA-3 (50 pMoles) and tryptic fragments of CM-NG LFA-3 (5-50 pMoles) to automated Edman degradation using an Applied Biosystems 470A gas phase sequencer in the presence of polybrene (Hewick et al., JBC, 256, p. 7990 (1981)). We identified PTH-amino acids on-line using an Applied Biosystems 120A PTH analyzer. Sequencing of the intact protein yielded a sequence of 38 amino acids as shown in Figure 1. Four tryptic fragments, T₇₂₋₇₃, T₉₁, T₁₀₅, and T₆₈ yielded the other DNA sequences, which are shown in Figure 1.

### Synthesis of Oligonucleotide Probes

We chemically synthesized two pools of anti-sense oligonucleotide DNA probes coding for regions from the amino terminal sequence of LFA-3 characterized by minimal nucleic acid degeneracy (see underscoring in Figure 1) on an Applied Biosystems 30A DNA synthesizer. For each selected amino acid sequence, we synthesized pools of probes complementary to all possible codons. We synthesized the probes anti-sense to enable hybridization of them to the corresponding sequences in DNA as well as in mRNA. We labelled our oligonucleotide probes using [γ-³²P]-ATP and polynucleotide kinase (Maxam and Gilbert, Proc. Natl. Acad. Sci., 74, p. 560 (1977)).

As depicted in Figure 2, the oligonucleotide probe pool LF1 was a 20-mer with 32-fold degeneracy. Probe pool LF2-5, was a 20-mer with 384-fold degeneracy. However, to reduce its degeneracy, we synthesized this pool in four subpools of 96-fold degeneracy each by splitting the degenerate codon for Gly into one of its four possible nucleotides for each subpool. We then selected the subpool containing the correct sequence from the three pools containing incorrect sequences by hybridization of the individual subpools to Northern blots containing human tonsil mRNA, as described previously (Wallner et al., Nature, 320, pp. 77-81 (1986)). Oligonucleotide probe subpool LF2 hybridized to a 1300 nucleotide transcript in human tonsil RNA, which suggested that it contained the correct sequence. Hence, we used it and pool LF1 for screening our various libraries.

### Construction of λgt10 Peripheral Blood Lymphocytes cDNA Library

To prepare our Peripheral Blood Lymphocytes (PBL) DNA library, we processed PBL from leukophoresis #9 through one round of absorption to remove monocytes. We then stimulated the non-adherent cells with IFN-γ 1000 U/ml and 10 µg/ml PHA for 24 h. We isolated RNA from these cells using phenol extraction (Maniatis et al., Molecular Cloning, p. 187 (Cold Spring Harbor Laboratory) (1982)) and prepared poly A⁺ mRNA by one round of oligo dT cellulose chromatography. We ethanol precipitated the RNA, dried it in a speed vac and resuspended the RNA in 10 µl H₂O (0.5 µg/µl). We treated the RNA for 10 min at room temperature in CH₃HgOH (5mM final concentration) and β-mercaptoethanol (0.26 M). We then added the methyl mercury treated RNA to 0.1 M Tris-HCl (pH 8.3) at 43°C, 0.01 M Mg, 0.01 M DTT, 2 mM Vanadyl complex, 5 µg oligo dT₁₂₋₁₈, 20 mM KCl, 1 mM dCTP, dGTP, dTTP, 0.5 mM dATP, 2 µCi[α-³²P]dATP and 30 U 1.5µl AMV reverse transcriptase (Seikagaku America) in a total volume of 50 µl. We incubated the mixture for 3 min at room temperature and 3 h at 44°C after which time we stopped the reaction by the addition of 2.5 µl of 0.5 M EDTA.

We extracted the reaction mixture with an equal volume of phenol:chloroform (1:1) and precipitated the aqueous layer two times with 0.2 volume of 10 M NH₄AC and 2.5 volumes EtOH and dried it under vacuum. The yield of cDNA was 1.5 µg.

We synthesized the second strand according to the methods of Okayama and Berg (Mol. Cell. Biol., 2, p. 161 (1982)) and Gubler and Hoffman (Gene, 25, p. 263 (1983)), except that we used the DNA polymerase I large fragment in the synthesis.

We blunt ended the double-stranded cDNA by resuspending the DNA in 80 µl TA buffer (0.033 M Tris Acetate (pH 7.8); 0.066 M KAcetate; 0.01M MgAcetate; 0.001M DTT; 50 µg/ml BSA), 5 µg RNase A, 4 units RNase H, 50 µM β NAD , 8 units E.coli ligase, 0.3125 mM dATP, dCTP, dGTP, and dTTP, 12 units T₄ polymerase and incubated the reaction mixture for 90 min at 37°C, added 1/20 volume of 0.5M EDTA, and extracted with phenol:chloroform. We chromatographed the aqueous layer on a G150 Sephadex column in 0.01M Tris-HCl (pH 7.5), 0.1 M NaCl, 0.001 M EDTA and collected the lead peak containing the double-stranded cDNA and ethanol precipitated it. Yield: 605 µg cDNA.

We ligated the double-stranded cDNA to linker 35/36 
5'AATTCGAGCTCGAGCGCGGCCGC3'
3'    GCTCGAGCTCGCGCCGGCG5'
 using standard procedures. We then size selected the cDNA for 800 bp and longer fragments on a S500 Sephacryl column, and ligated it to EcoRI digested λgt10. We packaged aliquots of the ligation reaction in Gigapak (Strategene) according to the manufacturer's protocol. We used the packaged phage to infect E.coli BNN102 cells and plated the cells for amplification. The resulting library contained 1.125x10⁶ independent recombinants.

### Screening of the Libraries

We screened human tonsil λgt10 cDNA library (Wong et al., Proc. Natl. Acad. Sci., 82, p. 7711-775 (1985)) and the PBL cDNA library prepared above with our labelled oligonucleotide probe LF1 using the plaque hybridization screening technique of Benton and Davis (Science, 196, p. 180 (1977)).

We pelleted an overnight culture of BNN102 cells in L broth and 0.2% maltose and resuspended it in an equal volume of SM buffer (50 mM Tris-HCl (pH 7.5), 100 mM NaCl, 10 mM MgSO₄, and 0.01% gelatin). Thereafter, we preabsorbed 9 ml of cells with 1.5x10⁶ phage particles at room temperature for 15 min and plated them on 30 LB Mg plates.

After incubation at 37°C for 8 h, we lifted the filters from the plates and lysed them by placing them onto a pool of 0.5 N NaOH/1.5 M NaCl for 5 min, and then submerged them for 5 min in the same buffer. We neutralized the filters by submerging them in 0.5 M Tris-HCl (pH 7.4), 1.5 M NaCl, two times for 5 min each, and rinsed them for 2 min in 1 M NH₄OAc, air dried the filters, and baked them for 2 h at 80°C.

We prehybridized and hybridized the filters to oligonucleotide probe LF1 in 0.2% polyvinylpyrolidone, 0.2% ficoll (MW 400,000), 0.2% bovine serum albumin, 0.05 M Tris-HCl (pH 7.5), 1 M sodium chloride, 0.1% sodium pyrophosphate, 1% SDS, 10% dextran sulfate (MW 500,000) and 100 µg/ml tRNA. We detected the hybridizing λ-cDNA sequences by autoradiography.

We initially selected 26 positive phages from the PBL library and 12 positive phages from the tonsil library. We then rescreened these clones and plaque purified them at lower density using the same probe.

We digested isolated DNA from these clones with EcoRI and hybridized them to oligomer probes LF1 and LF2 using the Southern blot technique (E. M. Southern, J. Mol. Biol., 98, pp. 503-18 (1975)). We then further characterized the DNA from two clones of the tonsil library (λHT16 and λHT12) and two clones from the PBL library (λP26 and λP24) by DNA sequencing analysis.

### Sequencing of cDNA Clones

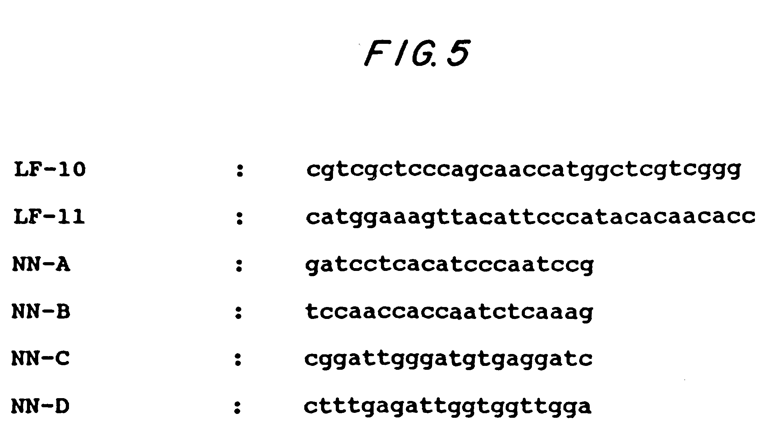

We subcloned the EcoRI digested DNA from clones λHT16 and λP26 into vector pNN01 to facilitate sequence analysis.* λHT16 has 2 EcoRI fragments which we individually subcloned into pNN01 resulting in plasmids pHT16H and pHT16L. The entire insert of λP26 is contained on a single NotI fragment. For subcloning, we used the vector's EcoRI site or SmaI site employing techniques in common use.
* We constructed sequencing plasmid pNN01 by removing the synthetic polylinker of pUC8 by restriction digestion and replacing it with a new synthetic segment. The 2.5 kb backbone common to the pUC plasmids, which provides an origin of replication and confers ampicillin resistance, is unchanged. The novel synthetic portion of pNN01 is shown in Figure 4.

We determined the DNA sequences of our subclones largely by the method of Maxam and Gilbert (Meth. Enzymology, 1980). However, for some fragments, we used the related procedure of Church and Gilbert (Proc. Natl. Acad. Sci. USA, 81, p. 1991 (1984)). The structure of pNN01 enables sequencing, by the Church-Gilbert approach, of the ends of an inserted fragment using NotI digestion and four 20-nucleotide long probes: NN-A, NN-B, NN-C and NN-D. See Figure 5.

Two sequencing results of significance were obtained directly from our subclones by the Church-Gilbert method. In one experiment, we probed PvuII digested DNA from λHT16 independently with LF10 and LF11 (Figure 5) to determine the sequence across the EcoRI sites bounding the 224 bp EcoRI fragment. This directly refuted the possibility that the analysis of the two EcoRI subclones (pHT16H and pHT16L) of this phage had overlooked sequences either 5' to the small EcoRI fragment or between the two EcoRI fragments. We also confirmed this result by analysis of the NotI subcloned pP26, which has this EcoRI site intact. We also digested pP26 with PvuII and sequenced the fragment by the Church-Gilbert method using LF10 (Fiqure 5) as hybridization probe. This proved that the 5' terminus of the insert of was exactly the same as that of λHT16.

Figure 3 shows the DNA sequence of the cDNA insert of phage λHT16. It also depicts the DNA sequence coding for LFA-3 and the amino acid sequence deduced therefrom. This latter cDNA, e.g., that coding for LFA-3, contained DNA sequences coding for the amino acid sequences that we had determined for the N-terminal region and the 4 tryptic fragments of LFA-3 purified from human erythrocytes.

### Expression of LFA-3 HT16 cDNA in CHO cells

960 bp NcoI-MaeI fragment of p24HT16LFA3#8 described below is inserted into the SmaI site of BG312 expresssion vector to give BG8 expression vector. Vector BG8 has been deposited with In Vitro International Culture Collection, 611 P. Hammonds Ferry Rd., Linthicum, Maryland, 21090 on May 24, 1988 and has been assigned accession number IVI-10170. In the BG312 (Cate et al., cell, 45, p.685 (1986)) the expression of the inserted DNA sequence is under the control of the Adenovirus 2 late promoter. p24HT16LFA3#8 was constructed by ligating a 800 bp EcoRI fragment of pHT16 with the large EcoRI fragment of p24, containing the plasmid portion. The DNA sequence of the EcoRI fragment of p24 corresponds to the DNA sequence of the base pairs 1-223 of figure 3. To establish a stable HT16-CHO cell line, we cotransfected 1 x 10⁷ CHO (DHFR⁻) cells (Chasin and Urlaub, Proc. Natl. Acad. Sci., 77, p. 4216 (1980)) with 180 µg of XmnI linearized BG8 DNA, 20 µg of StuI linearized pAdD26 DNA (Kaufman and Sharp, Mol. Cell. Biol., 2, p. 1304 (1982)) and 200 µg of sonicated salmon sperm DNA carrier by electroporation using a BIORAD (Richmond, California) gene pulser at 0.29 UV with capacitance set at 960 µFD. We next plated the electroporated CHO cells in non-selective alpha+ (modified Eagle Medium (MEM) for 3 days, then changed to alpha-MEM medium for 4 days at a cell density of 1x10⁵ cells per 100mm plate. Cells were then grown in alpha- MEM plus either 200 nM, 400 nM or 600 nM methotrexate. Colonies grew best in 200 nM methotrexate, from which we isolated individual clones, grew them to 1x10⁶ cells per 100 mm plate in order to assay for expression of LFA-3 by measuring the intensity of indirect immunofluorescence on a Fluorescence Activated Cell Sorter (FACS) (Becton Dickinson, Mountainview, California) and for rosetting (cell adhesion) as described below.

To analyze by FACS, 1 x 10⁶ cells per each HT16-CHO methotrexate clone and control CHO cells were removed from the tissue culture dishes by incubation with Hank's BSS buffer, .5 M EDTA at 4°C for 15 minutes. The detached cells were then pelleted, resuspended in 50 µl of PBN buffer (1 x PBS, .5% BSA, .1% sodium azide) and incubated with 100 µl of monoclonal antibody TS2/9 (1.2 mg/ml) (a gift of Tim Springer) on ice for 45 minutes. We next washed the cells two times with 1 ml PBN buffer and pelleted by centrifugation . The cell pellets were resuspended in 100µl of a 1:50 dilution of FCI (Fluorescein Conjugated Affinity Purified F (ab') 2 Fragment Sheep Anti-Mouse IgG (Cappel, Biomedical, Pennsylvania)) in PBN buffer and incubated on ice for 30 minutes. Cells were pelleted by centrifugation and excess FCI was removed by resuspending the cell pellets twice in 1 ml PBN buffer. We then resuspended the cells in 800 µl of 1 x PBS and determined the fluorescence intensity on FACS. Five clones showed higher fluorescence than control CHO cells and a homogeneous cell population with respect to LFA-3 expression.

We next tested whether the recombinant LFA-3 expressed in CHO cells could adhere to CD2 expressing cells by rosetting analysis. We grew control CHO cells and CHO cells expressing LFA-3 (HT16-CHO #30) in a 9.6 cm² well of a 6 well tissue culture plate at a cell density of 3 x 10⁵ cells per well. After washing the wells twice with Roswell Park Memorial Institute (RPMI 1060) medium to remove cell debris and dead cells, 3 x 10⁷ Jurkatt cells (a gift of Tim Springer) were added per well. Plates were spun at 400 rpm for 2 min. in a Sorvall Centrifuge at 4°C. After the cells were incubated at 4°C for 2 hours the wells were washed with RPMI 1060 medium to remove excess Jurkatt cells. Jurkatt cells rosetted with the LFA-3 expressing RT16-CHO #30 cells as determined under the microscope.

We compared the level of surface expression of LFA-3 in HT16-CHO #30 with the expression levels of JY B lymphoblastoid cells (a gift of. Tim Springer) by comparing the relative fluorescence intensities of both cell lines using FACS analysis as described above, using 2.5 x 10⁶ JY cells, CHO cells and HT16-CHO #30 cells. HT16-CHO cells showed a 6 fold higher fluorescence intensity than JY cells.

### Expression of LFA-3 HT16 cDNA in R1.1 Cells

2.6 x 10⁷ R1.1 cells were cotransfected with 90 µg NruI linearized expression vector BG8 as described above, 10µg NruI linearized plasmid pTCF DNA (F. Grosveld et al. Nucleic Acid Res., 10, p. 6715 (1982) and 300 µg sonicated salmon sperm DNA by electroporation (280 volts, 960 µFD) as described above. After selecting transfected cells in RPMI 1060 plus 1mg-ml G418, we isolated clones by dilution to 10³ cells per well, in a 96 well microtiter plate, containing 200 µl RPMI 1060 medium plus 1 mg/ml G418. Eight resistant clones were propagated and assayed for LFA-3 surface expression by FACS analysis as described above. We found all eight HT16-R1.1 clones expressed LFA-3 at levels approximately 25 fold above R1.1 controls, but all showed heterogeneous cell populations with regard to LFA-3 expression.

We further sorted two clones--#13 and #15--for high expression on FACS. 1 x 10⁶ HT16-R1.1 cells of each clone were incubated with 100 µl TS2/9 Mab for 45 minutes on ice in PBN buffer (1x PBS, 0.5% BSA, 0.1% sodium azide). After pelleting the cells we washed them twice by resuspending in 1 ml PBN buffer and resuspended the cell pellet in 100 µl of a 1:50 dilution of FCI. After 30 minutes on ice, the cells were washed and resuspended in 800 µl 1 x PBS. The surface expression of LFA-3 in the sorted homogeneous population of HT16-R1.1 #13 and #15 is 10% of that on JY cells.

We next tested whether LFA-3 from HT16 cDNA as expressed in R1.1 cells would adhere to other cells by rosetting with L-cells expressing CD2 cDNA (L114) (a gift of Catherine Hession) and with Jurkatt cells (a gift of Tim Springer) using the same method as described above for HT16-CHO. We observed rosetting. This rosetting could be inhibited with Mab to LFA-3 (TS2/9) or Mab to CD2 (TS2/18) (a gift of Tim Springer). This indicated that LFA-3 is expressed on the cell surface of R1.1 cells in a conformation that allows interaction with naturally expressed CD2 receptor on Jurkatt cells or with CD2 expressed on mouse L-cells. HT16-R1.1 cells or untransfected R1.1 cells do not rosette with untransfected mouse L-cells, indicating the specificity of these cellular interactions.

### Expression of LFA-3 HT16 cDNA in L-Cells

We cotransfected 1 x 10⁷ L (tk-) cells (C. P. Terhorst, J. Immun., 131, p. 2032 (1983)) with 90 µg of XmnI linearized BG8 plasmid DNA described above, 10 µg ScaI linearized pOPF plasmid DNA (Grosveld et al., supra) (a plasmid carrying the thymidine kinase gene which can be used to select transfected cells), and 300µg sonicated salmon sperm DNA by electroporation as described above. After washing and transferring the cells to 100mm petri dishes we grew them in nonselective medium (DMEM) for 48-72 hours. The transfected cells were then selected for in DMEM + hypoxanthine aminopterine thymidine (HAT) at a cell density of 1 x 10⁵ cells per 100 mm dish. We picked 13 clones expressing thymidine kinase after 14 days and expanded them to 1 x 10⁶ per 100 mm plate and assayed for LFA-3 expression by FACS analysis as described above. Eleven HT16-L clones showed a 10 to 20 fold higher surface fluorescence than control CHO cells.

We also assayed HT16-L clones for LFA-3 surface expression by immunofluorescence visualized directly under a fluorescence microscope. We found four clones -- HT16L #73, 1, 7,and 27 --were highly fluorescent, indicating high levels of LFA-3 expression. We next sorted all four clones for high expressing cells on the FACS as described above because these clones showed heterogeneous cell populations of LFA-3 expression. Six clones --HT16-L #1A, 1B, 1C, 7, 27, and 107 -- selected in HAT-DMEM medium were assayed for LFA-3 expression by FACS analysis. We found all clones showed fluorescence intensities of 20 to 30 fold over that of control CHO cells, except for clone HT16-L-1C which was 35 fold higher.

We also tested whether LFA-3 from HT16 cDNA as expressed in L-cells would adhere to other cells using the same rosetting assay described above for LFA-3 expressed in R1.1 cells. We observed rosetting.

### Expression of LFA-3 in E.coli

To express LFA-3 cDNA in E.coli, the expression vector pLFA3trcl was constructed. This vector (a derivative of pKK233-2) expresses the mature LFA-3 protein. The DNA sequence of HT16 cDNA coding for the signal sequence was deleted. To construct pLFA3trcl, plasmid pKK233-2 (E. Aman and J. Brofius Gene, 40 p. 183 (1985)) was digested with restriction enzymes NcoI and HindIII, and ligated to an isolated 700 base pair Ava2/Hind3 fragment of pLFA3HT16 and a double stranded linker LF21-22. This linker replaces the sequence between base pairs 101 to 175 (to Ava2 site) of HT16 cDNA (Table I).

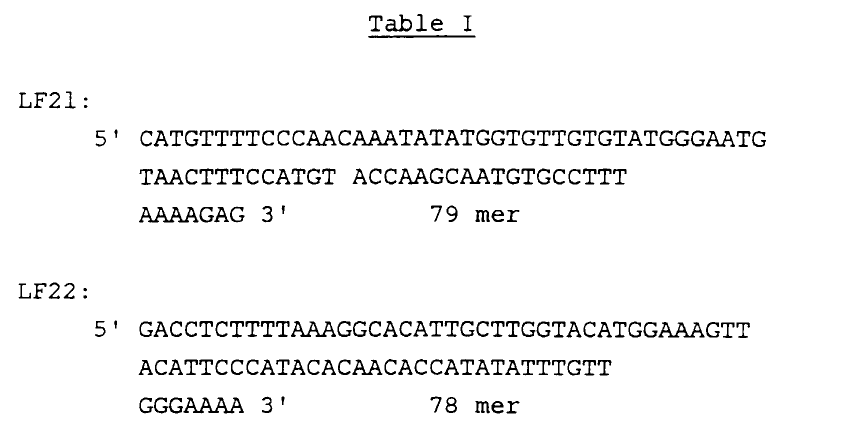

We transformed JA221 cells with the resulting recombinant expression vector. E. coli transformed with pLFA3trcl were grown to OD₅₅₀ 1.0, followed by inducing with lmM IPTG for 2 hours and collecting the cells by centrifugation. Cells were resuspended in LB and an equivalent of 0.2 OD₅₅₀ was removed. We next pelleted by centrifugation, resuspended the cell pellet in 50 ul SDS-gel loading buffer and boiled for 3 minutes. 25 ul of the sample was loaded onto a SDS-denaturing polyacrylamide gel in order to separate the proteins by electrophoresis. To detect LFA-3, proteins were electrotransferred onto a nitrocellulose membrane, followed by incubating the membrane with a 1:1000 dilution of an antibody to h-LFA-3, K64 (a gift of Tim Springer). We observed that the anti-LFA-3 (K64) antibodies detected a 25 kd protein, indicating that the transformed cells expressed LFA-3.

Cells were next grown in 1 1 LB + 50 pg/ml ampicillin at 37°C to OD₅₅₀ 0.1. Expression of LFA-3 was induced with 2 mM IPTG for 2 hours. Cells were pelleted at 4000 rpm/4°C for 30 minutes. We resuspended the pellets in 25 mM Tris (pH 7.0) at 1 gm cell pellet per 10 ml Tris buffer and lysed in a French Pressure Cell Press at cell pressure 12,000-14,000 psi. Membrane fractions were next separated from soluble proteins by centrifugation in SS34 Sorvall at 10,000 rpm for 30 minutes at 4°C. Membrane pellets were resuspended in 1 ml of 25 mM Tris pH 7.0. 1 µl aliquots of the supernatant and of the membrane pellets were electrophoresed on a 15% SDS-denaturing PAG. The membrane pellets were dissolved in SDS buffer and the proteins were separated by SDS-PAGE, coomassie stained and the LFA-3 excised from the gel for subsequent protein sequencing. Our amino acid sequencing confirmed the N-terminal sequence as M F S Q Q I Y G V V Y G N V T-LFA-3 and F S Q Q I Y G V V Y G N V T-LFA-3 which matches the N-terminal sequence determined for the human LFA-3 purified from human erythrocytes (Wallner, et al. J. Exp. Med., 166:923 (1987)). Accordingly, the product produced by this construction has a N-terminal with and without methionine.

We also modified the DNA sequence encoding LFA-3 to delete from it portions of the hydrophobic transmembrane domain to obtain high levels of expression of soluble forms of LFA-3. We used our LFA-3 cDNA from pHT16 as depicted in Figure 3 which codes for LFA-3 containing a transmembrane domain.

### LFA-3M17 Deletion

50 µg of plasmid pHT16LFA3 DNA was digested with 100 units of the restriction enzyme ScaI for 1 hour at 37°C. We separated the linearized plasmid from the uncut plasmid by electrophoresis on a 1% agarose gel, excised it from the gel and electroeluted. Another aliquot of 50 µg of pHT16LFA3 plasmid DNA was digested with 100 units of EcoRI and 100 units of Hind III for 1 hour at 37°C to excise most of the LFA-3 cDNA sequences in this plasmid. The vector was next separated from the EcoRI/HdIII fragment by electrophoresis on a 1% agarose gel, excised and electroeluted.

We used an oligonucleotide LF17, to introduce the deletion. This is a 43 mer (depicted in Table II) corresponding to 21 nucleotides, starting at base pairs 632 and 22 nucleotides starting at position 764 base pairs of the HT16 cDNA sequence depicted in Figure 3.

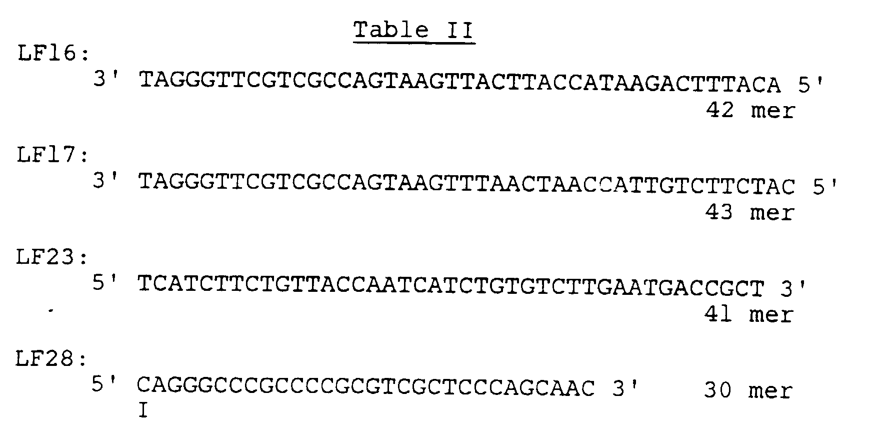

When this oligonucleotide is hybridized to single-stranded HT16 cDNA, it will loop out 111 base pairs of the HT16 cDNA. 20 pmoles of the electroeluted ScaI and 20 pmoles of the EcoRI/HindIII fragments were mixed with 20 pmoles of phosphorylated LF17, followed by denaturing in 100 mM NaCl, 6 mM Tris (pH 7.6) and 8 mM MgCl₂ at 90°C for 5 minutes and renaturing by incubation at 37°C for 1 hour, at 4°C for 1 hour, and on ice for 10 minutes. To one half of the reaction mix, we added 0.5 µl T4 ligase, 1 mM ATP, 1 µl Klenow and 5 mM of NTP, and the mixture was incubated at 15°C overnight. One half of the reaction mix was transformed into MC1061 competent cells. Colonies containing deleted LFA-3 cDNA were identified by hybridization to LF17, as follows. Transformed MC1061 colonies were transferred to nitrocellulose filters, lysed and the DNA denatured by treatment with 0.5 N NaOH. The nitrocellulose filters were hybridized in PSB containing 1x10⁵ cpm/ml ³²P-kinased oligonucleotide LF17 at 65°C overnight. Filters were washed at 65°C in 0.1 x SSC (.015M sodium chloride, .0015 M sodium citrate) and exposed to X-ray film. Positive colonies were picked and grown at 37°C, and lysed. We next prepared DNA (see, Maniatis et al., "Molecular Cloning: A Laboratory Manual", Cold Spring Harbor (1982)). To verify the correct deletion, DNA was digested with EcoRI and Hind III. Four pLFA3M17 colonies had the correct restriction pattern. The correct DNA sequence of one clone was confirmed by DNA sequencing using the method of Maxam and Gilbert (supra).

### LFA-3M16

Plasmid pLFA3M16-2-4H was constructed essentially as described for LFA3M17, except that the oligonucleotide LF16 (Table II) was used to introduce the deletion. LF16 is a 42 mer of which 21 nucleotides are homologous to base pairs 632 to 652 (Figure 3) and 21 nucleotides to base pairs 716 to 736 (Figure 3) of HT16 cDNA. Mutagenesis with oligonucleotide LF16 deleted the 63 base pair sequence coding for the hydrophobic potential transmembrane domain of LFA-3, leaving the cytoplasmic domain intact. We isolated plasmid pLFA3M16, and verified its correct sequence as described above.

### LFA-3M23

Plasmid pLFA3M23 was constructed by procedures described for pLFA3M17. The oligonucleotide used for the mutagenesis was LF23 (Table II), a 41 mer of which twenty-one nucleotides are homologous to base pairs 641 to 661 (Figure 3) and 20 nucleotides to base pairs 767 to 786 (Figure 3) of HT16 cDNA. Mutagenesis with LF23 deleted the potential transmembrane and cytoplasmic domain of HT16-LFA-3 cDNA.

We next inserted all deleted LFA-3 cDNAs into expression vectors that also carry a selective marker. We believe having the selective marker on the same plasmid in close proximity to the LFA-3 gene during integration will assure cointegration of these two genes and, therefore, their coselection. We used the expression vector pJOD-s. Vector pJOD-s has been deposited with In Vitro International, Inc. Culture Collection in Linthicum, Maryland, on May 26, 1988, and has been assigned accession number IVI-10171. For the construction of LFA-3M17, M16 and M23; the pJOD-s vector was linearized with restriction enzyme SalI, blunt-ended with Klenow and ligated with the Klenow-blunt ended NotI fragment of pLFA3M17, pLFA3M16 or pLFA3M23. The respective ligation mixtures were next transformed into MC1061, and colonies containing the respective DNA were selected by hybridization to oligonucleotide probe LF28 (see Table II), a 30 mer, homologous to base pairs 20 to 49 of HT16 cDNA at 65°C in PSB and washed at 0.5 x SSC (0.75M NaCl, 0.075M NaCl) at 65°C. Positive colonies were picked, grown overnight in LB + amp 50 pg/ml, and DNA prepared. The correct sizes of the inserts were verified by restriction enzyme mapping. Four clones of pLFA3M17, three clones of pLFA3M16 and one clone of pLFA3M23 showed the correct restriction enzyme patterns. DNA was prepared from a clone of pLFA3M17, pLFA3M16 and pLFA3M23, and the correct DNA sequences were confirmed by DNA sequencing using the method of Maxam and Gilbert (supra).

To establish stable CHO cell lines, 10 µg of pLFA3M17 that had been linearized with PvuI was transfected into CHO cells by the calcium phosphate procedure and electroporation as described above. The same procedure is used for establishing stable CHO cell lines with pLFA3M16 and pLFA3M23.

CHO cells transfected with pLFA3M17 were assayed for secretion of soluble LFA-3 by metabolic labeling with ³⁵S methionine. 5x10⁵ cells per well of a 6 well plate were labeled with ³⁵S methionine for 18 hours and ³⁵S-LFA-3 precipitated with Mab TS2/9. We observed that M17/CHO secreted LFA-3. It should be understood that to obtain higher expression, M17/CHO may be amplified in high concentrations of methotrexate to amplify the LFA-3 gene.

To enable further the above-described invention, we deposited the following phage carrying an LFA-3 DNA sequence of this invention in the In Vitro International, Inc. Culture Collection in Linthicum, Maryland, on May 28, 1987:

λHT16 [λgt10/LFA-3]

The phage has been assigned accession number IVI 10133.

While we have herein before presented a number of embodiments of this invention, it is apparent that our basic construction can be altered to provide other embodiments which utilize the processes and compositions of this invention. Therefore, it will be appreciated that the scope of this invention is to be defined by the claims appended hereto rather than the specific embodiments which we have presented by way of example.

## Claims

1. A DNA sequence selected from the group consisting of:
(a) the DNA insert carried in phage λHT16 (ATCC 75107) - formerly IVI 10133);
(b) DNA sequences which hybridize under conditions equivalent to about 20° to 27° below Tm and 1 M sodium chloride to the LFA-3 DNA sequence of (a) and which code on expression for a polypeptide that binds to CD2, the receptor on the surface of T-lymphocytes; and
(c) DNA sequences which are degenerate to any of the DNA sequences of (a) and (b) and code on expression for the same polypeptides.

2. The DNA sequence according to claim 1, wherein said DNA sequence (b) codes on expression for a polypeptide that binds to CD2 on the surface of T-lymphocytes and inhibits adhesion between T-lymphocytes and target cells.

3. The DNA sequence according to claim 2, wherein said polypeptide is soluble.

4. The DNA sequence according to claim 1, wherein said DNA sequence is selected from the group consisting of a DNA sequence of the formula N₁₋₁₀₄₇ of Figure 3, a DNA sequence of the formula N₁₇₋₇₆₆ of Figure 3, a DNA sequence of the formula N₁₀₁₋₇₆₆ of Figure 3, a DNA sequence of the formula N₁₀₁₋₆₁₁-N₇₁₆₋₇₆₆ of Figure 3, a DNA sequence of the formula ATG-N₁₀₁₋₇₆₆ of Figure 3, a DNA sequence of the formula ATG-N₁₀₁₋₆₆₁-N₇₁₆₋₇₆₆ of Figure 3, a DNA sequence of the formula N₁₋₆₅₂-N₇₁₆₋₁₀₄₇ of Figure 3, and DNA sequence of the formula N₁₋₆₅₂-N₇₆₄₋₁₀₄₇ of Figure 3, a DNA sequence of the formula N₁₋₆₆₁-N₇₆₄₋₁₀₄₇, and DNA sequences that are degenerate to any of the above DNA sequences.

5. The DNA sequence according to claim 1, wherein said DNA sequence is selected from the group consisting of a DNA sequence of the formula N₁₇₋₇₆₆ of Figure 3, a DNA sequence of the formula ATG-N₁₇₋₇₆₆ of Figure 3, and DNA sequences that are degenerate to any of the above DNA sequences.

6. A recombinant DNA molecule comprising a DNA sequence according to claim 1, 2, 3, 4 or 5 operatively linked to an expression control sequence in said recombinant DNA molecule.

7. The recombinant DNA molecule according to claim 6, wherein said expression control sequence is selected from the group consisting of the early or late promoters of SV40 or adenovirus, the lac system, the trp system, the TAC system, the TRC system, the major operator and promoter regions of phage λ, the control regions of fd coat protein, the promoter for 3-phosphoglycerate kinase or other glycolytic enzymes, the promoters of acid phosphatase and the promoters of the yeast α-mating factors.

8. The recombinant DNA molecule according to claim 6, wherein the molecule is BG8 (ATCC 68791- formerly IVI 10170).

9. A unicellular host transformed with a recombinant DNA molecule comprising a DNA sequence according to claim 1, 2, 3, 4 or 5 operatively linked to an expression control sequence in said recombinant DNA molecule.

10. The host according to claim 9, wherein said host is selected from the group consisting of strains of E.coli, Pseudomonas, Bacillus, Streptomyces, fungi, animal cells, plant cells and human cells in tissue culture.

11. The unicellular host according to claim 10, wherein the animal cell is selected from the group consisting of CHO, R1.1 and L-M(tK⁻).

12. A polypeptide encoded by a DNA sequence selected from the group consisting of the DNA sequences of claims 1 to 5, said polypeptide being essentially free of other proteins of human origin, characterised in that said polypeptide has a formula selected from the group consisting of: 
M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R Y A L I P I P L A V I T T C I V L Y M N G I L
K C D R K P D R T N S N;

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R Y A L I P I P L A V I T T C I V L Y M N G I L
K C D R K P D R T N S N;

M F S Q Q I Y G V V Y G N V T F H V P S N V P L K E V L W K
K Q K D K V A E L E N S E F R A F S S F K N R V Y L D T V S
G S L T I Y N L T S S D E D E Y E M E S P N I T D T M K F F
L Y V L E S L P S P T L T C A L T N G S I E V Q C M I P E H
Y N S H R G L I M Y S W D C P M E Q C K R N S T S I Y F K M
E N D L P Q K I Q C T L S N P L F N T T S S I I L T T C I P
S S G H S R H R Y A L I P I P L A V I T T C I V L Y M N G I
L K C D R K P D R T N S N;

F S Q Q I Y G V V Y G N V T F H V P S N V P L K E V L W K K
Q K D K V A E L E N S E F R A F S S F K N R V Y L D T V S G
 
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R M N G I L K C D R K P D R T N S N;

M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S M N G I L K C D R K P D R T N S N;

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S M N G I L K C D R K P D R T N S N;

M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S;

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S;
 
M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R;
 and

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R.

13. A method of producing an LFA-3 polypeptide comprising culturing a unicellular host according to claim 9.

14. The method according to claim 12, wherein the transformed host is selected from the group consisting of CHO(BG8), R1.1(BG8) and L-M(tK⁻) (BG8), wherein BG8 is identified by deposit number ATCC 68791- formerly IVI 10170.

15. A pharmaceutical composition comprising an immunosuppressant or enhancement effective amount of a polypeptide selected from the group consisting of the polypeptides of claim 12 and a pharmaceutically acceptable carrier.

16. A diagnostic composition to detect, in vitro, CD2⁺ cells, such as T-cell subsets, or to monitor in vitro the course of diseases characterized by excess or depleted CD2⁺ T-cells comprising a diagnostic effective amount of a polypeptide selected from the group consisting of the polypeptides of claim 12.

17. An in vitro method of detecting CD2⁺ cells, such as T-cell subsets, or for monitoring the course of diseases characterized by excess or depleted CD2⁺ T-cells comprising the step of employing, as a diagnostic, a composition selected from the group consisting of the compositions of claim 16.

18. A means for detecting, in vitro, CD2⁺ cells, such as T-cell subsets, or for monitoring in vitro the course of diseases characterized by excess or depleted CD2⁺ T-cells comprising a composition selected from the group consisting of the compositions of claim 16.

## Patentansprüche

1. Eine DNS Sequenz, ausgewählt aus der Gruppe bestehend aus:
(a) dem DNS-Zentralabschnitt, der enthalten oder getragen ist in dem Phagen λHT16 (ATCC 75107 - zuvor IVI 10133);
(b) DNS Sequenzen, die unter Bedingungen entsprechend ungefähr 20 bis 27° unter Tm und 1 Mol Natriumchlorid hybridisieren zu der LFA-3 DNS Sequenz von (a) und welche bei der Expression ein Polypeptid kodieren, welches an CD2, den Rezeptor an der Oberfläche von T-Lymphozyten, bindet; und
(c) DNS Sequenzen, die degeneriert sind zu irgendeiner der DNS Sequenzen von (a) und (b) und die bei der Expression dasselbe Polypeptid kodieren.

2. Die Sequenz gemäss Anspruch 1, wobei die DNS-Sequenz (b) eine Expression für ein Polypeptid kodiert, welches an CD2 an der Oberfläche von T-Lymphozyten bindet, und welches die Verbindung zwischen T-Lymphozyten und Zielzellen hemmt.

3. Die DNS Sequenz gemäss Anspruch 2, wobei das Polypeptid lösbar ist.

4. Die DNS Sequenz gemäss Anspruch 1, wobei die DNS Sequenz ausgewählt ist aus der Gruppe bestehend aus einer DNS Sequenz der Formel N₁₋₁₀₄₇ von Figur 3, einer DNS Sequenz der Formel N₁₇₋₇₆₆ von Figur 3, einer DNS Sequenz der Formel N₁₀₁₋₁₇₆₆ von Figur 3, einer DNS Sequenz der Formel N₁₀₁₋₆₁₁-N₇₁₆₋₇₆₆ von Figur 3, einer DNS Sequenz der Formel ATG-N₁₀₁₋₇₆₆ von Figur 3, einer DNS Sequenz der Formel ATG-N₁₀₁₋₆₆₁-N₇₁₆₋₇₆₆ von Figur 3, einer DNS Sequenz der Formel N₁₋₆₅₂-N₇₁₆₋₁₀₄₇ von Figur 3, und einer DNS Sequenz der Formel N₁₋₆₅₂-N₇₁₆₋₁₀₄₇ von Figur 3, einer DNS Sequenz der Formel N₁₋₁₆₆-N₇₆₄₋₁₀₄₇, und DNS Sequenzen, die degeneriert sind zu irgeneiner der obigen DNS Sequenzen.

5. Die DNS Sequenz gemäss Anspruch 1, wobei die DNS Sequenz ausgewählt ist aus der Gruppe, bestehend aus einer DNS Sequenz der Formel N₁₇₋₇₆₆ von Figur 3, einer DNS Sequenz der Formel ATG-N₁₇₋₇₆₆ von Figur 3 und DNS Sequenzen, die degeneriert sind zu irgendeiner der obigen DNS Sequenzen.

6. Ein rekomprimiertes DNS Molekül, umfassend oder bestehend aus DNS Sequenzen gemäss Ansprüchen 1, 2, 3, 4 oder 5, operativ gebunden an eine Expressions-Steuer-Sequenz in dem rekomprimierten DNS Molekül.

7. Das rekombinierte DNS Molekül gemäss Anspruch 6, wobei die Expressions-Steuer-Sequenz ausgewählt ist aus der Gruppe, bestehend aus den frühen oder späten Promotern von SV40 oder Adenovirus, des lac-Systems, des trp-Systems, des TAC-Systems, des TRC-Systems, der hauptsächlichen Operator- und Promotor-Regionen von Phage λ, der Steuerregionen von fd-beschichtetem Protein, dem Promotor für 3-Phosphoglycerat-Kinase oder anderen glycolytischen Enzymen, den Promotern für Phosphatasesäure und den Promotern des Hefe-α-Verbindungs-Faktors.

8. Das rekombinierte DNS Molekül entsprechend Anspruch 6, wobei das Molekül BG8 ist (ATCC 68791 - vormals IVI 10170).

9. Einen einzelligen Wirt, umgeformt mit einem rekombinierten DNS Molekül, umfassend eine DNS Sequenz gemäss Anspruch 1, 2, 3, 4, oder 5, operativ gebunden an eine Expressions-Steuer-Sequenz in dem rekombinierten DNS Molekül.

10. Ein Wirt entsprechend Anspruch 9, wobei der Wirt ausgewählt ist aus der Gruppe, bestehend aus Strängen von E.coli, Pseudomonas, Bacillus, Streptomyces, Pilzen, tierischen Zellen, Pflanzenzellen und menschlichen Zellen in Gewebekultur.

11. Der unizellulare Wirt gemäss Anspruch 10, wobei die tierische Zelle ausgewählt ist aus der Gruppe, bestehend aus CHO, R1.1 und L-M (tK⁻).

12. Ein Polypeptid, kodiert durch eine DNS Sequenz, die ausgewählt ist aus der Gruppe, bestehend aus den DNS Sequenzen nach Ansprüchen 1 bis 5, wobei das Polypeptid im Wesentlichen frei ist von anderen Proteinen menschlichen Ursprunges, dadurch gekennzeichnet, dass das Polypeptid eine Formulierung hat, die ausgewählt ist aus der Gruppe bestehend aus: 
M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R Y A L I P I P L A V I T T C I V L Y M N G I L
K C D R K P D R T N S N;
 
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R Y A L I P I P L A V I T T C I V L Y M N G I L
K C D R K P D R T N S N;

M F S Q Q I Y G V V Y G N V T F H V P S N V P L K E V L W K
K Q K D K V A E L E N S E F R A F S S F K N R V Y L D T V S
G S L T I Y N L T S S D E D E Y E M E S P N I T D T M K F F
L Y V L E S L P S P T L T C A L T N G S I E V Q C M I P E H
Y N S H R G L I M Y S W D C P M E Q C K R N S T S I Y F K M
E N D L P Q K I Q C T L S N P L F N T T S S I I L T T C I P
S S G H S R H R Y A L I P I P L A V I T T C I V L Y M N G I
L K C D R K P D R T N S N;

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R M N G I L K C D R K P D R T N S N;

M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S M N G I L K C D R K P D R T N S N;
 
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S M N G I L K C D R K P D R T N S N;

M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S;

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S;

M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R;
 und

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R.

13. Ein Verfahren zur Herstellung eines LFA-3 Polypeptids, wobei ein einzelliger Wirt gemäss Anspruch 9 kultiviert wird.

14. Das Verfahren nach Anspruch 13, wobei der transformierte Wirt ausgewählt ist aus der Gruppe, bestehend aus CHO(BG8), R1.1(BG8) und L-M(tK⁻) (BG8), wobei BG8 identifiziert ist durch die Hinterlegungsnummer ATCC 68791 - vormals IVI 10170.

15. Eine pharmazeutische Zusammensetzung oder Gemisch, enthaltend einen immunrepressiven oder immunverstärkenden, wirksamen Anteil eines Polypeptids, ausgewählt aus der Gruppe, bestehend aus den Polypeptiden nach Anspruch 12 und einem pharmazeutisch akzeptablen Träger.

16. Eine Diagnose-Mischung zur In-Vitro-Erkennung von CD2⁺ Zellen, wie T-Zellen-Teilmengen (Untermengen), oder zur In-Vitro-Anzeige des Verlaufs von Krankheiten, gekennzeichnet durch Überschuss oder Mangel bis zur Erschöpfung von CD2⁺ T-Zellen, enthaltend einen diagnostisch wirksamen Betrag eines Polypeptids, ausgewählt aus der Gruppe, bestehend aus den Polypeptiden nach Anspruch 12.

17. Ein In-Vitro-Verfahren der Erkennung oder Feststellung von CD2⁺ Zellen, wie T-Zell-Teilmengen (Untermengen), oder zur Anzeige des Verlaufs von Krankheiten, gekennzeichnet durch Überschuss oder Mangel bis zur Erschöpfung von CD2⁺ T-Zellen, umfassend die Stufen der Anwendung als Diagnosemittel, einer Zusammensetzung ausgewählt aus der Gruppe, bestehend aus der Zusammensetzung nach Anspruch 16.

18. Ein Mittel zum In-Vitro-Nachweis von CD2⁺ Zellen, wie T-Zell-Teilmengen (Untermengen), oder zur Anzeige in vitro des Verlaufs von Krankheiten, gekennzeichnet durch Überschuss oder Mangel bis zur Erschöpfung von CD2⁺ T-Zellen, umfassend eine Zusammensetzung, ausgewählt aus der Gruppe, bestehend aus der Zusammensetzung nach Anspruch 16.

## Revendications

1. Séquence d'ADN choisie dans le groupe qui consiste en :
a) l'insert d'ADN porté dans le phage λHT16 (ATCC 75107), précédemment IVI 10133),
b) des séquences d'ADN qui hybrident dans des conditions équivalentes à environ 20 en 27° en dessous de Tm et le chlorure de sodium 1 M en la séquence ADN LFA-3 de a) et qui code à l'expression pour un polypeptide qui se lie à CD2, le récepteur à la surface des lymphocytes T, et
c) des séquences d'ADN qui sont dégénérées en toutes les séquences de a) et de b) et qui codent à l'expression pour les mêmes polypeptides.

2. Séquence d'ADN selon la revendication 1,
caractérisée en ce que
cette séquence d'ADN b) code à l'expression pour un polypeptide qui se lie au CD2 à la surface des lymphocytes T et qui inhibe l'adhésion entre les lymphocytes T et les cellules cible.

3. Séquence d'ADN selon la revendication 2,
caractérisée en ce que
ce polypeptide est soluble.

4. Séquence d'ADN selon la revendication 1,
caractérisée en ce que
cette séquence d'ADN est choisie dans le groupe qui consiste en une séquence d'ADN de formule N₁₋₁₀₄₇ selon la figure 3, une séquence d'ADN de formule N₁₇₋₇₆₆ selon la figure 3, une séquence d'ADN de formule N₁₀₁₋₇₆₆ selon la figure 3, une séquence d'ADN de formule N₁₀₁₋₆₁₁-N₇₁₆₋₇₆₆ selon la figure 3, une séquence d'ADN de formule ATG-N₁₀₁₋₇₆₆ selon la figure 3, une séquence d'ADN de formule ATG-N₁₀₁₋₆₆₁-N₇₁₆₋₇₆₆ selon la figure 3, une séquence d'ADN de formule N₁₋₆₅₂N₇₁₆₋₁₀₄₇ selon la figure 3, et une séquence d'ADN de formule N₁₋₆₅₂-N₇₆₄₋₁₀₄₇ selon la figure 3, une séquence d'ADN de formule N₁₋₆₆₁-N₇₆₄₋₁₀₄₇ et des séquences d'ADN qui sont dégénérées par rapport à n'importe laquelle des séquences d'ADN ci-dessus.

5. Séquence d'ADN selon la revendication 1,
caractérisée en ce que
cette séquence d'ADN est choisie dans le groupe qui consiste en une séquence d'ADN de formule N₁₇₋₇₆₆ selon la figure 3, une séquence d'ADN de formule ATG-N₁₇₋₇₆₆ selon la figure 3, et des séquences d'ADN qui sont dégénérées par rapport à n'importe laquelle des séquences d'ADN ci-dessus.

6. Molécule d'ADN recombinante qui comprend une séquence d'ADN selon les revendications 1, 2, 3, 4 ou 5, liée fonctionnellement à une séquence de contrôle de l'expression dans cette molécule d'ADN recombinante.

7. Molécule d'ADN recombinante selon la revendication 6,
caractérisée en ce que
la séquence de contrôle de l'expression est choisie dans le groupe qui consiste en les promoteurs précoces ou tardifs de SV40 ou d'adénovirus, le système lac, le système trp, le système TAC, le système TRC, l'opérateur principal et les régions du promoteur du phage λ, les régions de contrôle de la protéine d'enveloppe fd, le promoteur pour la 3-phosphoglycérate Kinase ou d'autres enzymes glycolytiques, les promoteurs de phosphatase acide et les promoteurs des facteurs d'accouplement en α de levure.

8. Molécule d'ADN recombinante selon la revendication 6,
caractérisée en ce que
la molécule est BG8 (ATCC 68791-précédemment IVI 10170).

9. Hôte unicellulaire transformé par une molécule d'ADN selon la revendication 1, 2, 3, 4 ou 5, reliée fonctionnellement à une séquence de contrôle de l'expression dans cette molécule d'ADN recombinante.

10. Hôte selon la revendication 9,
caractérisé en ce que
cet hôte est choisi dans le groupe qui consiste en des souches d'E. Coli, Pseudomonas, Bacillus, Streptomyces, des champignons, des cellules animales, des cellules de plante, et des cellules humaines en culture de tissu.

11. Hôte unicellulaire selon la revendication 10,
caractérisé en ce que
la cellule animale est choisie dans le groupe qui consiste en CHO, R1.1 et L-M-(tK⁻).

12. Polypeptide encodé par une séquence d'ADN choisie dans le groupe qui consiste en les séquences d'ADN des revendications 1 à 5, ce polypeptide étant essentiellement dépourvu d'autre protéine d'origine humaine,
caractérisé en ce que
ce polypeptide a une formule choisie dans le groupe qui consiste en : 
M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R Y A L I P I P L A V I T T C I V L Y M N G I L
K C D R K P D R T N S N;
 
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R Y A L I P I P L A V I T T C I V L Y M N G I L
K C D R K P D R T N S N;

M F S Q Q I Y G V V Y G N V T F H V P S N V P L K E V L W K
K Q K D K V A E L E N S E F R A F S S F K N R V Y L D T V S
G S L T I Y N L T S S D E D E Y E M E S P N I T D T M K F F
L Y V L E S L P S P T L T C A L T N G S I E V Q C M I P E H
Y N S H R G L I M Y S W D C P M E Q C K R N S T S I Y F K M
E N D L P Q K I Q C T L S N P L F N T T S S I I L T T C I P
S S G H S R H R Y A L I P I P L A V I T T C I V L Y M N G I
L K C D R K P D R T N S N;

F S Q Q I Y G V V Y G N V T F H V P S N V P L K E V L W K K
Q K D K V A E L E N S E F R A F S S F K N R V Y L D T V S G

S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R M N G I L K C D R K P D R T N S N;

M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S M N G I L K C D R K P D R T N S N;
 
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S M N G I L K C D R K P D R T N S N;

M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S;

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S;

M V A G S D A G R A L G V L S V V C L L H C F G F I S C
F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R;
 et

F S Q Q I Y G V V Y G N V T F H V P S N V P L K E V L W K K
Q K D K V A E L E N S E F R A F S S F K N R V Y L D T V S G
S L T I Y N L T S S D E D E Y E M E S P N I T D T M K F F L
Y V L E S L P S P T L T C A L T N G S I E V Q C M I P E H Y
N S H R G L I M Y S W D C P M E Q C K R N S T S I Y F K M E
N D L P Q K I Q C T L S N P L F N T T S S I I L T T C I P S
S G H S R H R.

13. Procédé de production d'un polypeptide LFA-3 qui comprend la mise en culture d'un hôte unicellulaire selon la revendication 9.

14. Procédé selon la revendication 13,
caractérisé en ce que
l'hôte transformé est choisi dans le groupe qui consiste en CHO(BG8), R1.1(BG8) et L-M-(tK⁻) (BG8), dans lequel BG8 est identifié par son numéro de dépôt ATCC 68791 - précédemment IVI 10170.

15. Composition pharmaceutique comprenant un immunosuppresseur ou une quantité efficace d'augmentation d'un polypeptide choisi dans le groupe qui consiste dans le polypeptide de la revendication 12 et un vecteur pharmaceutiquement acceptable.

16. Composition de diagnostic pour détecter, « in vitro », des cellules CD2 telles que des sous-groupes de cellules T ou pour surveiller « in vitro » le déroulement de maladies caractérisées par un excès ou un épuisement des cellules T CD2⁺ qui comprend une quantité efficace de diagnostic d'un polypeptide choisi dans le groupe des polypeptides de la revendication 12.

17. Procédé « in vitro » pour détecter des cellules CD2⁺ telles que des sous-groupes de cellules T, ou pour surveiller le déroulement de maladies caractérisées par un excès ou un épuisement des cellules T CD2⁺' qui comprend l'étape qui consiste à employer comme un diagnostic, une composition choisie dans le groupe formé des compositions de la revendication 16.

18. Moyen pour détecter « in vitro » des cellules CD2⁺ telles que des sous-groupes de cellules T, ou pour surveiller « in vitro » le déroulement de maladies caractérisées par un excès ou par un épuisement de cellules T CD2⁺ qui comprend une composition choisie dans le groupe formé des compositions de la revendication 16.
